# EUROPÄISCHE PATENTSCHRIFT

(11) **EP 3 723 595 B1**
(45) Veröffentlichungstag und Bekanntmachung des Hinweises auf die Patenterteilung: **29.04.2026**
(21) Anmeldenummer: 18833407.2
(22) Anmeldetag: 14.12.2018
(51) Int. Cl.: A61B 5/01, A61B 5/00, A61M 1/16, A61M 1/14, A61M 1/36

(54) **VERFAHREN UND VORRICHTUNG ZUR ERFASSUNG DER KÖRPERTEMPERATUR EINES PATIENTEN**
METHOD AND APPARATUS FOR ACQUIRING THE BODY TEMPERATURE OF A PATIENT
PROCÉDÉ ET DISPOSITIF POUR DÉTERMINER LA TEMPÉRATURE CORPORELLE D'UN PATIENT

(30) Priorität: 15.12.2017 DE 102017130107
(43) Veröffentlichungstag der Anmeldung: 21.10.2020
(73) Patentinhaber: Fresenius Medical Care Deutschland GmbH, 61352 Bad Homburg (DE)
(72) Erfinder: SPICKERMANN, Reiner, 97535 Burghausen (DE)
(74) Vertreter: Lorenz Seidler Gossel Part. mbB
(86) Internationale Anmeldenummer: PCT/EP2018/085010
(87) Internationale Veröffentlichungsnummer: WO 2019/115789

(56) Entgegenhaltungen:
- DE-A1- 102014 111 403
- DE-U1- 202012 005 708
- US-A1- 2003 171 691
- US-A1- 2009 080 757
- US-A1- 2012 212 455
- US-A1- 2014 121 845

## Beschreibung

Die vorliegende Erfindung betrifft ein Verfahren und eine Vorrichtung zur Erfassung der Körpertemperatur, vorzugsweise der Kerntemperatur des Körpers eines Patienten, wobei der Patient eine Fistel zur Abfuhr von Blut in einen extrakorporalen Kreislauf und/oder zur Zufuhr von Blut aus einem extrakorporalen Kreislauf aufweist.

Im Rahmen der Dialysebehandlung ist ein Patientenmonitoring von großer Bedeutung, um einen sicheren Behandlungsverlauf zu gewährleisten und um beim Eintritt unerwünschter Situationen bzw. Zustände des Patienten schnell und zielgerichtet eingreifen zu können. Ein wichtiger Patientenparameter, der während der Behandlung zu überwachen ist, ist die Körpertemperatur des Patienten.

Die Messung der Körpertemperatur des Patienten ermöglicht beispielsweise die Erkennung von Infektionen, ist aber auch im Rahmen der Wärmeregulation des Patienten während der Dialysebehandlung von großer Bedeutung. Dabei stellt insbesondere die Hämodialyse eine Herausforderung für die Wärmeregulation dar, da sich während der Behandlung die Temperatur des Blutes der Temperatur des Dialysats angleicht.

Üblicherweise wird das Blut dem Patienten in einem Temperaturgleichgewicht mit dem Dialysat zurückgeführt, wobei sich der Wärmeübergang von dem Blut zu dem Dialysat in dem Dialysator vollzieht, der einen Bestandteil des extrakorporalen Kreislaufs des Dialysegerätes bildet.

Je nachdem, welche Temperatur das Dialysat aufweist, kann es somit zum einem Anstieg oder Abfall der Körpertemperatur kommen, wobei bereits geringfügige Änderungen der Körpertemperatur (insbesondere der Kerntemperatur des Körpers), z.B. im Bereich von 0,3 - 0,8 °C komplexe Gegensteuerungsmechanismen des Körpers zur Folge haben. Diese Mechanismen dienen zur Konstanthaltung der Körpertemperatur in einem bestimmten Bereich. Die Gegensteuerungsmechanismen bzw. die Änderung der Körpertemperatur beeinflussen entweder die Kreislaufstabilität, z.B. durch Erweiterung der Gefäße oder Blutdruckabfall bei Überwärmung des Blutes, oder den Patientenkomfort, der sich z.B. in einem Kälteempfinden, Frösteln etc. beim Abkühlen des Blutes bemerkbar macht.

Vor diesem Hintergrund ist es von Wichtigkeit, die Körpertemperatur des Patienten während der Behandlung konstant oder im Wesentlichen konstant zu halten, d.h. den Patienten "isotherm" zu halten. Dies kann dadurch erreicht werden, dass die Bluttemperatur des Patienten überwacht und die Dialysattemperatur entsprechend angepasst wird.

Die Messung der Körpertemperatur kann beispielsweise mit einem handelsüblichen Fieberthermometer oder auch mittels eines Ohr- oder Stirnthermometes erfolgen.

Nachteilig bei dieser Messmethode ist es, dass je nach Ort der Messung (oral, rektal, Achselhöhle, Trommelfell) die Körpertemperatur nicht präzise erfasst werden kann, eine relativ lange Messdauer erforderlich ist, das Messergebnis durch die Umgebungsbedingungen beeinflusst wird und zudem Artefakten unterliegen kann. Im Übrigen ist es von Nachteil, dass Manipulationen am Patienten vorzunehmen sind, die von diesem ggf. als unkomfortabel empfunden werden oder insgesamt unpraktisch für den Ablauf der Dialysebehandlung sind.

Ein weiterer Nachteil dieser bekannten Messmethoden besteht darin, dass diese nicht kontinuierlich, sondern nur in zeitlichen Abständen durchgeführt werden können, dass also nur eine intermittierende Messung der Temperatur möglich ist.

Zwar kann eine kontinuierliche Temperaturmessung im Ösophagus des Patienten erfolgen, jedoch setzt diese ein invasives und für den Patienten entsprechend unangenehmes Verfahren voraus, bei dem ein Temperatursensor als Sonde in die Speiseröhre des Patienten eingebracht wird und dort für die Dauer der Messung verbleibt.

Die DE102014111403A1 offenbart ein Verfahren zur Überwachung der Ausrichtung und/oder Positionierung einer mit einer arteriellen und/oder venösen Patientennadel verbundenen Blutschlauchleitung, oder wenigstens eines Abschnitts der Blutschlauchleitung, in einem Zugangsbereich eines extrakorporalen Blutkreislaufs.

Die vorgenannten Messverfahren sind für eine Dialysebehandlung vor allem deshalb nachteilig, weil die Messung mit Verzögerungen verbunden ist und Änderungen der Körpertemperatur somit nur mit einer Verzögerung wiedergegeben werden bzw. erfasst werden können. Dies schließt eine sehr schnelle Reaktion des Gerätes bzw. des Personals auf unerwünschte Temperaturänderungen des Patienten aus bzw. erschwert diese.

Eine schnelle Temperaturmessung des Blutes ist mittels eines Infrarotsensors am Blutschlauch des extrakorporalen Kreislaufs möglich. Dieses Verfahren ist jedoch technisch aufwändig und kostenintensiv.

Der vorliegenden Erfindung liegt die Aufgabe zugrunde, die Nachteile des Stands der Technik zu beheben und insbesondere ein verbessertes Verfahren und eine verbesserte Vorrichtung zur Erfassung der Körpertemperatur, vorzugsweise der Körperkerntemperatur, bereitzustellen.

Diese Aufgabe wird durch ein Verfahren mit den Merkmalen des Anspruchs 1 und eine Vorrichtung mit den Merkmalen des Anspruch 7 gelöst.

Danach ist vorgesehen, dass zum Zwecke der Erfassung der Körpertemperatur des Patienten mittels einer Infrarot-Kamera eine Wärmebild-Aufnahme, im Folgenden auch einfach als "Wärmebild" bezeichnet, der Fistel oder eines Teils der Fistel aufgenommen wird. Der vorliegenden Erfindung liegt somit der Gedanke zugrunde, eine aus dem Stand der Technik bekannte Wärmebildmessung mittels Infrarotkamera auf die Oberfläche der Fistel bzw. der Haut im Bereich der Fistel anzuwenden. Unter "Erfassung der Körpertemperatur" ist nicht zwingend zu verstehen, dass ein Temperaturwert, wie z.B. 37 °C gemessen wird. Der Begriff umfasst auch die Feststellung eines für die Temperatur repräsentativen Wertes, wie z.B. die Farbe des Wärmebildes im Bereich der Fistel etc.

Aus dem auf diese Weise entstandenen Wärmebild der Fistel bzw. der Haut über der Fistel kann auf die Körperkerntemperatur, auf die Hauttemperatur, auf die Bluttemperatur oder auf eine sonstige Körpertemperatur des Patienten geschlossen werden.

Unter einer Fistel wird vorzugsweise eine nicht natürliche, sondern operativ eingesetzte Verbindung zwischen einer Arterie und einer Vene des Patienten verstanden. Diese kann aus einem natürlichen oder künstlichen Gewebe bestehen.

Der Begriff "Körpertemperatur" ist weit zu verstehen und umfasst die Temperatur an jeder beliebigen Stelle des Körpers, wie z.B. auf der Hautoberfläche, im Blut etc.

Vorzugsweise ist unter dem Begriff der "Körpertemperatur" die "Körperkerntemperatur" zu verstehen.

Die Aufnahme des Wärmebildes erfolgt vorzugsweise kontaktlos, d.h. ohne den Patienten zu berühren, so dass dieser in keiner Weise beeinträchtigt wird. Die Aufnahme ist vergleichsweise einfach durchzuführen und zudem genau. Die unterschiedlichen Farben bzw. Farbtiefen repräsentieren die jeweils vorliegenden Temperaturen.

Die Messung bzw. Aufnahme kann ohne großen technischen Aufwand erfolgen und ist komfortabel für den Patienten. Hochpreisige Bauteile und technisch besonders komplexe Bauteile sind nicht erforderlich.

Die Aufnahme des Wärmebildes kann kontinuierlich vorgenommen werden oder auch in bestimmen oder unregelmäßigen Zeitabständen, z.B. bei Eintritt besonderer Ereignisse, die eine Überprüfung der Körpertemperatur als notwendig erscheinen lassen.

In einer bevorzugten Ausgestaltung der Erfindung kann mittels der Wärmebild-Aufnahme die Temperatur der Haut des Patienten gemessen werden, d.h. auf einen konkreten Temperaturwert geschlossen werden. Die Umsetzung bzw. "Umrechnung" des Wärmebildes auf Temperaturwerte ist jedoch kein zwingendes Erfordernis der Erfindung. Grundsätzlich ist es auch denkbar, dass die gemessene Farbe im Bereich der Fistel mit einem Referenzwert bzw. mit einer Referenzfarbe verglichen wird und basierend darauf die Feststellung getroffen wird, ob sich die Temperatur des Patienten in einem Sollbereich befindet oder nicht.

Vorzugsweise erfolgt die Aufnahme bzw. die Temperaturmessung im infraroten Spektralbereich (IR) mit einem Array, d.h. einem zweidimensionalen Messfeld.

Vorzugsweise wird die maximale Temperatur auf der IR-Fläche ermittelt. Durch die zweidimensionale Messung und die Ermittlung der maximalen Temperatur werden Bewegungen des Arms des Patienten mit erfasst und beeinflussen damit das Messergebnis nicht.

Vorteilhaft ist es, dass die Oberflächentemperatur der Haut des betroffenen Arms weitgehend mit der Fistelinnentemperatur übereinstimmt, so dass von der ermittelten Hauttemperatur auf die Fisteltemperatur und damit auf die Bluttemperatur geschlossen werden kann. Ggf. kann die Erfassung der Fistelinnentemperatur zu Beginn der Behandlung empirisch oder durch Abgleich mit der Hauttemperatur präzisiert werden.

Die auf diese Weise ermittelte Fistelinnentemperatur kann mit der Körperkerntemperatur gleichgesetzt werden. Geht man von einem Herzminutenvolumen von 5 l/min aus, ist der Fluss in der Fistel typischerweise 1 l/min und damit sehr hoch. Dies lässt den Schluss zu, dass die Fistelinnentemperatur identisch oder nahezu (± 5 %) identisch mit der Körperkerntemperatur ist. Damit kann auf einen Algorithmus zur Bestimmung der Körperkerntemperatur verzichtet werden.

Die Aufnahme des Wärmebilds und/oder die darauf basierende Messung der Körpertemperatur kann vor und/oder nach einer Dialysebehandlung durchgeführt werden.

Wie oben ausgeführt, kann die Wärmebild-Aufnahme kontinuierlich oder intermittierend genommen werden. Dies gilt für die Ableitung der Körpertemperatur basierend auf der Wärmebild-Aufnahme entsprechend.

Denkbar ist es weiterhin, dass ein Istwert der Wärmebild-Aufnahme oder einer daraus abgeleiteten Größe mit einem Sollwert oder Sollwertbereich verglichen wird und dass eine Regelung vorgenommen wird, so dass der Istwert mit dem Sollwert übereinstimmt oder in dem Sollwertbereich liegt. Auf diese Weise ist eine Regelung der Körpertemperatur möglich. Diese Regelung der Körpertemperatur kann beispielsweise über eine Veränderung des Durchflusses und/oder der Temperatur des Dialysats erfolgen, das den Dialysator auf der Dialysatseite durchströmt.

Diese Regelung wird vorzugsweise von einer Regelungseinheit des Dialysegerätes durchgeführt.

Auch andere Stellgrößen sind denkbar und von der Erfindung mit umfasst.

Bei der Regelgröße kann es sich im Rahmen der Erfindung um die aus dem Wärmebild gewonnene Temperatur oder auch um einen anderen Parameter, beispielsweise um die Farbe des Wärmebildes handeln.

Um dem Patienten bzw. dem medizinischen Personal einen Anhaltspunkt für die Temperatur des Patienten geben zu können, ist es denkbar, dass eine Abweichung zwischen Ist- und Sollwert oder Sollwertbereich z.B. numerisch angezeigt wird, dass somit eine entsprechende Anzeigevorrichtung, z.B. am Dialysegerät vorgesehen ist.

Die vorliegende Erfindung betrifft des Weiteren eine Vorrichtung in Form eines Dialysegerätes, wie z.B. ein Hämodialysegerät, ein Hämofiltrationsgerät oder ein Hämodiafiltrationsgerät mit einem extrakorporalen Blutkreislauf, in dem sich ein Dialysator befindet, der im Betrieb des Gerätes von Blut des Patienten durchströmt wird, wobei das Dialysegerät eine Wärmebildkamera aufweist, die geeignet und ausgerichtet ist, von der Fistel oder einem Teil der Fistel des an dem Dialysegerät angeschlossenen Patienten eine Wärmebild-Aufnahme anzufertigen.

Die Wärmebildkamera kann sich z.B. an der Infusionsstange des Dialysegerätes befinden und/oder einen integralen Bestandteil des Dialysegerätes bilden.

Die Wärmebildkamera kann beispielsweise als Infrarot-Pistole oder durch ein sonstiges mobiles Gerät gebildet werden, was den Vorteil mit sich bringt, dass ein Arzt von einem Behandlungsplatz zum nächsten gehen kann und schnell und einfach Wärmebildaufnahmen vornehmen kann.

Erfindungsgemäß ist der Aufnahmebereich der Wärmebildkamera veränderbar, so dass dieser beispielsweise der Bewegung des Arms des Patienten und somit auch der Bewegung der Fistel nachgeführt wird.

Dieses Nachführen erfolgt automatisch, d.h. durch das Dialysegerät, indem dieses die Wärmebildkamera einem Referenzpunkt nachführt. Dieser Referenzpunkt verändert seine Lage relativ zur Fistel nicht. Bei dem Referenzpunkt handelt sich um die wärmste Stelle des Armes in dem durch die Wärmebildkamera aufgenommenen Bereich.

Das Dialysegerät kann einen Regler aufweisen, mittels dessen die Temperatur in der Fistel oder im Bereich der Fistel auf einem Sollwert oder in einem Sollwertbereich gehalten werden kann. So ist es möglich, z.B. die Körperkerntemperatur des Patienten während einer Dialysebehandlung auf einem Sollwert oder in einem Sollwertbereich zu halten. Diese Regelung wird von dem Dialysegerät vorzugsweise selbsttätig durchgeführt, so dass kein Eingriff des Nutzers notwendig ist. Vorzugsweise ist vorgesehen, dass der Dialysator im Betrieb des Gerätes dialysatseitig von Dialysat durchströmt wird und dass der Regler derart ausgeführt ist, dass als Stellgrößen die Temperatur und/oder die Durchflussrate des Dialysats durch den Regler veränderbar sind. Durch die Temperatur und/oder Durchflussrate des Dialysats kann die Temperatur des den Dialysator blutseitig durchströmenden Blutes konstant gehalten, gesteigert oder gesenkt werden. Der Dialysator umfasst vorzugsweise eine Membran oder eine Vielzahl von Membranen, vorzugsweise Hohlfasermembranen, die die Blutseite von der Dialysatseite trennen und über die ein Stoff- und Wärmeübergang möglich ist.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine Wärmebildaufnahme zweiter Unterarme, wobei in dem rechts dargestellten Arm eine Fistel angeordnet ist und
- Figur 2:: eine Wärmebildaufnahme des in Figur 1 rechts dargestellten Arms mit einer Temperaturangabe in der Fistel.

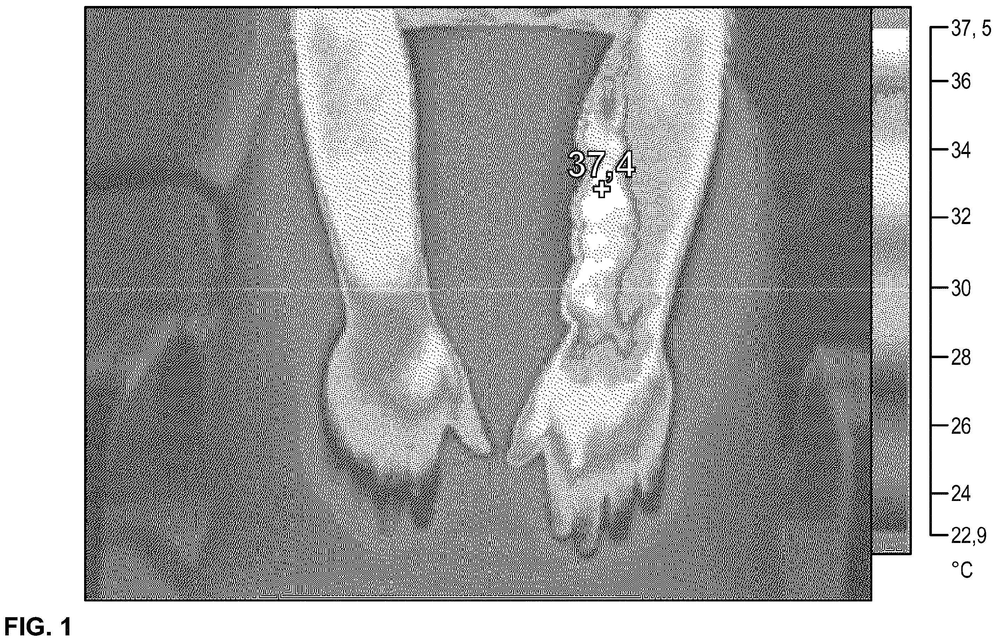

Aus Figur 1 ist eine Wärmbildaufnahme zweiter Unterarme eines Patienten erkennbar, wobei die unterschiedlichen Farben bzw. Grautöne für unterschiedliche Temperaturen stehen. Rechts von Figur 1 ist eine Temperaturskala angegeben, die eine Zuordnung der Farben bzw. der Grautöne zu Temperaturwerten ermöglicht.

Aus Figur 1 ist ersichtlich, dass der rechts dargestellte Arm in dem mit dem Kreuz gekennzeichneten Bereich, in dem sich die Fistel befindet, die größte Temperatur aufweist.

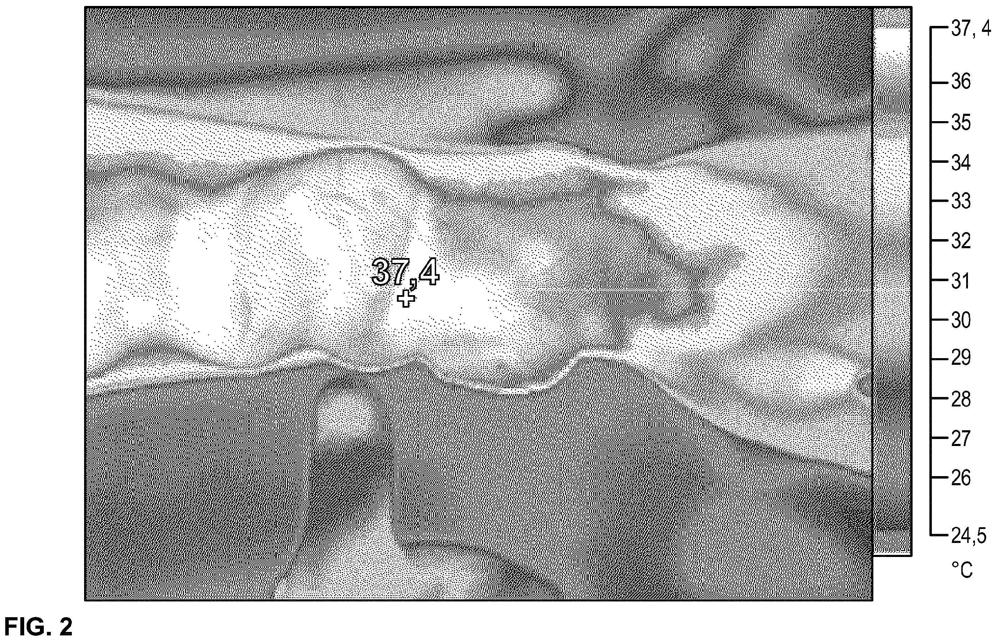

Dies ergibt sich auch aus der vergrößerten Darstellung gemäß Figur 2. Aus dieser ergibt sich, dass für die Fistel und somit letztlich auch für das durch die Fistel fließende Blut und damit auch für die Körperkerntemperatur eine Isttemperatur von 37,4 °C ergibt.

Dieser Wert liegt in einem Sollwertbereich, so dass keine Maßnahmen zur Temperaturänderung zu treffen sind.

Ist die Temperatur des Blutes in der Fistel jedoch zu gering, kann deren Erhöhung z.B. durch eine Erwärmung des Dialysats erreicht werden, das den Dialysator des extrakorporalen Kreislauf dialysatseitig durchströmt. Ist die gemessene Temperatur zu hoch, kann entsprechend eine Verringerung der Bluttemperatur durch eine Verringerung der Temperatur des Dialysats erreicht werden.

Durch die vorliegende Erfindung ist es vergleichsweise einfach möglich Rückschlüsse auf die Kerntemperatur eines Patienten zu ziehen. Basierend darauf ist es möglich, diese auf einen Sollwertbereich zu regeln, um sicherzustellen, dass der Patient während der Dialysebehandlung nicht gefährdet wird und sich während der Behandlung wohl fühlt. Aufwendige oder gar invasive Apparaturen zur Messung der Körpertemperatur sind nicht erforderlich.

## Patentansprüche

1. Verfahren zur Erfassung der Körpertemperatur eines Patienten mittels eines Dialysegeräts gemäß einem der Ansprüche 7 bis 11, wobei der Patient eine Fistel zur Abfuhr von Blut in einen extrakorporalen Kreislauf und/oder zur Zufuhr von Blut aus einem extrakorporalen Kreislauf aufweist, wobei zum Zwecke der Erfassung der Körpertemperatur des Patienten mittels einer Infrarot-Kamera eine Wärmebild- Aufnahme der Fistel oder eines Teils der Fistel angefertigt wird, dass der Bereich der maximalen Temperatur identifiziert wird und dass eine Veränderung der Position dieses Bereiches bei der Erfassung bzw. Auswertung der Wärmebild-Aufnahme berücksichtigt wird, wobei ein Aufnahmebereich der Infrarot-Kamera dem Bereich der maximalen Temperatur nachgeführt wird, wobei die Körpertemperatur vor oder nach einer Dialysebehandlung gemessen wird.

2. Verfahren nach Anspruch 1, **dadurch gekennzeichnet, dass** mittels der Wärmebild-Aufnahme die Temperatur der Haut des Patienten erfasst wird und/oder dass von diesem erfassten Temperaturwert der Haut auf die Bluttemperatur in der Fistel und/oder auf die Körperkerntemperatur geschlossen wird.

3. Verfahren nach einem der vorhergehenden Ansprüchen, **dadurch gekennzeichnet, dass** die Wärmebild-Aufnahme kontinuierlich oder intermittierend aufgenommen wird.

4. Verfahren nach einem der vorhergehenden Ansprüche, **dadurch gekennzeichnet, dass** die Messung der Körpertemperatur basierend auf der Wärmebild-Aufnahme kontinuierlich oder intermittierend durchgeführt wird.

5. Verfahren nach einem der vorhergehenden Ansprüche, **dadurch gekennzeichnet, dass** die Wärmebild-Aufnahme in einem zweidimensionalen Array aufgenommen wird.

6. Verfahren nach einem der vorhergehenden Ansprüche, **dadurch gekennzeichnet, dass** die Wärmebild-Aufnahme durch eine Infrarot-Pistole oder durch ein sonstiges mobiles Gerät aufgenommen wird.

7. Dialysegerät mit einem extrakorporalen Blutkreislauf, in dem sich ein Dialysator befindet, der im Betrieb des Gerätes von Blut eines eine Fistel aufweisenden Patienten durchströmt wird, wobei das Dialysegerät eine Wärmebildkamera aufweist, die geeignet und ausgerichtet ist, von der Fistel oder von einem Teil der Fistel des Patienten eine Wärmebild-Aufnahme anzufertigen, wobei das Dialysegerät weiterhin dazu ausgelegt ist, den Bereich der maximalen Temperatur zu identifizieren und eine Veränderung der Position dieses Bereiches bei der Erfassung bzw. Auswertung der Wärmebild-Aufnahme zu berücksichtigen und einen Aufnahmebereich der Infrarot-Kamera dem Bereich der maximalen Temperatur als Referenzpunkt nachzuführen.

8. Dialysegerät nach Anspruch 7, **dadurch gekennzeichnet, dass** sich die Wärmebildkamera an der Infusionsstange des Dialysegerätes befindet und/oder einen integralen Bestandteil des Dialysegerätes bildet und/oder dass der Aufnahmebereich der Wärmebildkamera veränderbar ist.

9. Dialysegerät nach Anspruch 7 oder 8, **dadurch gekennzeichnet, dass** das Dialysegerät Bestimmungsmittel aufweist, mittels derer aus der Wärmebild-Aufnahme ein Temperaturwert ermittelbar ist.

10. Dialysegerät nach einem der Ansprüche 7 bis 9,
**dadurch gekennzeichnet,**
**dass** das Dialysegerät einen Regler aufweist, mittels dessen die Temperatur der Haut über der Fistel oder die Temperatur in der Fistel oder im Bereich der Fistel auf einem Sollwert oder in einem Sollwertbereich gehalten werden kann.

11. Dialysegerät nach Anspruch 10, **dadurch gekennzeichnet, dass** der Dialysator im Betrieb des Gerätes dialysatseitig von Dialysat durchströmt wird und dass der Regler derart ausgeführt ist, dass als Stellgrößen zum Zwecke der Regelung die Temperatur und/oder die Durchflussrate des Dialysats durch den Regler veränderbar sind.

## Claims

1. Method for detecting the body temperature of a patient by means of a dialysis machine according to one of claims 7 to 11, wherein the patient has a fistula for removing blood into an extracorporeal circuit and/or for supplying blood from an extracorporeal circuit, wherein, for the purpose of detecting the body temperature of the patient by means of an infrared camera, a thermal image of the fistula or of a part of the fistula is produced, the region of maximum temperature is identified, and a change in the position of this region is taken into account during the acquisition and/or evaluation of the thermal image, wherein an imaging region of the infrared camera is tracked to follow the region of maximum temperature, wherein the body temperature is measured before or after a dialysis treatment.

2. Method according to claim 1, **characterized in that**, by means of the thermal image, the temperature of the patient's skin is detected and/or that, from this detected temperature value of the skin, the blood temperature in the fistula and/or the core body temperature is inferred.

3. Method according to one of the preceding claims, **characterized in that** the thermal image is recorded continuously or intermittently.

4. Method according to one of the preceding claims, **characterized in that** the measurement of the body temperature based on the thermal image is carried out continuously or intermittently.

5. Method according to one of the preceding claims, **characterized in that** the thermal image is recorded in a two-dimensional array.

6. Method according to one of the preceding claims, **characterized in that** the thermal image is recorded by an infrared gun or by another mobile device.

7. Dialysis machine having an extracorporeal blood circuit, in which a dialyzer is located, through which, during operation of the machine, blood of a patient having a fistula flows, wherein the dialysis machine comprises a thermal imaging camera which is suitable and oriented to produce a thermal image of the fistula or of a part of the fistula of the patient, wherein the dialysis machine is furthermore designed to identify the region of maximum temperature and to take into account a change in the position of this region during the acquisition and/or evaluation of the thermal image and to track an imaging region of the infrared camera to follow the region of maximum temperature as a reference point.

8. Dialysis machine according to claim 7, **characterized in that** the thermal imaging camera is located on the infusion pole of the dialysis machine and/or forms an integral component of the dialysis machine and/or that the imaging region of the thermal imaging camera is variable.

9. Dialysis machine according to claim 7 or 8, **characterized in that** the dialysis machine has determination means by means of which a temperature value can be determined from the thermal image.

10. Dialysis machine according to one of claims 7 to 9, **characterized in that** the dialysis machine has a controller by means of which the temperature of the skin above the fistula or the temperature in the fistula or in the region of the fistula can be maintained at a setpoint value or within a setpoint range.

11. Dialysis machine according to claim 10, **characterized in that**, during operation of the machine, the dialyzer is flowed through on the dialysate side by dialysate and that the controller is designed such that, for the purpose of control, the temperature and/or the flow rate of the dialysate can be varied by the controller as manipulated variables.

## Revendications

1. Procédé de détection de la température corporelle d'un patient au moyen d'un appareil de dialyse selon l'une des revendications 7 à 11, le patient présentant une fistule pour l'évacuation de sang vers un circuit extracorporel et/ou pour l'alimentation en sang à partir d'un circuit extracorporel, une image thermique de la fistule ou d'une partie de la fistule étant réalisée, à l'aide d'une caméra infrarouge, aux fins de la détection de la température corporelle du patient, la zone de température maximale étant identifiée et une modification de la position de cette zone étant prise en compte lors de l'acquisition et/ou de l'évaluation de l'image thermique, une zone de prise de vue de la caméra infrarouge étant asservie à la zone de température maximale, la température corporelle étant mesurée avant ou après un traitement de dialyse.

2. Procédé selon la revendication 1, **caractérisé en ce que**, au moyen de l'image thermique, la température de la peau du patient est détectée et/ou **en ce que**, à partir de cette valeur de température détectée de la peau, on déduit la température du sang dans la fistule et/ou la température centrale du corps.

3. Procédé selon l'une des revendications précédentes, **caractérisé en ce que** l'image thermique est acquise de manière continue ou intermittente.

4. Procédé selon l'une des revendications précédentes, **caractérisé en ce que** la mesure de la température corporelle sur la base de l'image thermique est réalisée de manière continue ou intermittente.

5. Procédé selon l'une des revendications précédentes, **caractérisé en ce que** l'image thermique est acquise dans une matrice bidimensionnelle.

6. Procédé selon l'une des revendications précédentes, **caractérisé en ce que** l'image thermique est acquise au moyen d'un pistolet infrarouge ou d'un autre dispositif mobile.

7. Appareil de dialyse comportant un circuit sanguin extracorporel, dans lequel se trouve un dialyseur, qui, pendant le fonctionnement de l'appareil, est traversé par le sang d'un patient présentant une fistule, l'appareil de dialyse comportant une caméra thermique qui est apte et orientée à réaliser une image thermique de la fistule ou d'une partie de la fistule du patient, l'appareil de dialyse étant en outre conçu pour identifier la zone de température maximale et pour prendre en compte une modification de la position de cette zone lors de l'acquisition et/ou de l'évaluation de l'image thermique, et pour asservir une zone de prise de vue de la caméra infrarouge à la zone de température maximale en tant que point de référence.

8. Appareil de dialyse selon la revendication 7, **caractérisé en ce que** la caméra thermique se trouve sur la potence de perfusion de l'appareil de dialyse et/ou constitue une partie intégrante de l'appareil de dialyse et/ou **en ce que** la zone de prise de vue de la caméra thermique est modifiable.

9. Appareil de dialyse selon la revendication 7 ou 8, **caractérisé en ce que** l'appareil de dialyse comporte des moyens de détermination au moyen desquels une valeur de température peut être déterminée à partir de l'image thermique.

10. Appareil de dialyse selon l'une des revendications 7 à 9, **caractérisé en ce que** l'appareil de dialyse comporte un régulateur au moyen duquel la température de la peau au-dessus de la fistule ou la température dans la fistule ou dans la zone de la fistule peut être maintenue à une valeur de consigne ou dans une plage de valeurs de consigne.

11. Appareil de dialyse selon la revendication 10, **caractérisé en ce que** le dialyseur, pendant le fonctionnement de l'appareil, est traversé, du côté dialysat, par du dialysat et **en ce que** le régulateur est conçu de telle sorte que, aux fins de la régulation, la température et/ou le débit du dialysat peuvent être modifiés par le régulateur en tant que grandeurs de réglage.
